# EUROPEAN PATENT APPLICATION

(11) **EP 4 349 241 A1**
(43) Date of publication of application: **10.04.2024**
(21) Application number: 22315230.7
(22) Date of filing: 05.10.2022
(51) Int. Cl.: A61B 1/00, A61B 1/005, A61M 25/01, A61B 17/34

(54) **INSERTION INSTRUMENT, SYSTEM COMPRISING THE INSERTION INSTRUMENT AND PROCESS FOR MANUFACTURING THE INSERTION INSTRUMENT**

(71) Applicant: Caranx Medical SAS, 75013 Paris (FR)
(72) Inventor: BERTHET-RAYNE, Pierre, 06800 Cagnes-sur-Mer (FR); PRZYBYLSKI, Flavie, 06800 Cagnes sur Mer (FR); SAHAFI, Ali, 5230 Odense M (DK)
(74) Representative: Hepp Wenger Ryffel AG

(57) **Abstract**

The invention relates to an insertion instrument (101) for insertion into a cavity (41) the instrument (101) comprising a flexible tubular sheath (2) having a proximal annular end (21), a distal annular end (22), and a middle section (25) which extends between the proximal annular end (21) and the distal annular end (22), the insertion instrument (101) is adapted for reversibly everting a collapsed internal section (23) of the sheath (2) to an expanded external section (24) of the sheath (2) at the distal annular end (22) such that a length (4) of the middle section (25) increases in a longitudinal direction of the tubular sheath (2), wherein the insertion instrument (101) comprises at least one of a shape sensor (5, 6, 7, 8, 10) for detecting a shape of at least a part of the sheath (2), a position sensor (5, 6, 7, 8, 10) for detecting the spatial position of at least a part of the sheath (2) relative to the cavity (41), and an environment sensor (5, 6, 7, 8, 10) for detecting a distance of a part of the sheath (2) relative to the cavity (41) or for detecting a property of the cavity (41), which is/are configured for acquiring status data of the sheath (2) and/or the cavity (41).

## Description

The invention relates to an insertion instrument for insertion into a cavity, a system comprising the insertion instrument, and a process for manufacturing an insertion instrument for insertion into a cavity.

Minimally invasive surgical interventions, in particular in the intragastric tract or vascular system, require advancing a medical device along a body lumen to a target site for carrying out the surgical procedure.

Recently, evertible growing robots have been used to perform these interventions because the mass of the evertible growing robot does not need to move with respect to its environment, such as a gastric lumen. This allows the evertible growing robot to be safely moved forward in the environment without causing increased friction but instead only everting an inner section to a radially expanded outer section at its distal end.

US 6,554,793 B1 discloses a flexible surgical trocar assembly having an invertible drive tube and a drive mechanism contacting the inner invertible drive tube for moving an inner wall of the trocar in a proximal or distal direction.

US 2019/217908 A1 discloses a robot for navigating through an environment by growing which controls the shape of its body by actively controlling the length of wall material provided by its distal end during growth.

Recent advances of growing robots as a new low-cost technology configured to be used in harsh inaccessible environments, for example narrow body ducts or intragastric organs, show an increased need for avoiding damage to surrounding tissue or collision with structures of the patient's body. This requires particular skills of the operator and/or external imaging.

It is the object of the present invention to overcome these and other disadvantages of the prior art. The invention shall provide an insertion instrument, in particular a surgical insertion instrument, for insertion into a cavity or lumen, preferably a human cavity which allows a safe and well controllable insertion.

Status data, which enable the insertion instrument to be placed precisely in a target site/identifying the target site, might be particularly helpful for that purpose.

The selection and placement of sensors is particularly difficult due to the evertible nature of such an insertion instrument.

Consequently, the choice of sensors, data to be collected and way of data collection should be optimized and adapted to the nature of the growing robot with an essentially immobile outer section with respect to the body lumen while being everted.

The prior art fails to provide a sensor which provides valuable status data of an evertible insertion instrument as such and/or cavity, e.g. to detect a shape of the insertion instrument/cavity or a force exerted on the insertion instrument, to classify tissue, in particular pathologies, or reconstruct a three-dimensional shape of the insertion instrument/cavity. These status data provide improved safety features to a patient, allow to control deformations of the insertion instrument, and can be used to optimize guidance of the insertion instrument to a specific target site.

The insertion instrument comprises a flexible tubular sheath having a proximal annular end, a distal annular end, and a middle section which extends between the proximal annular end and the distal annular end.

The insertion instrument optionally comprises a pressurizing unit for pressurizing at least one inflatable pressure tight chamber of the flexible tubular sheath, in particular at or within the distal annular end. The pressurizing unit then comprises a fluid supply which is fluid-connected or connectable to the inflatable chamber for supplying a pressurized fluid.

The insertion instrument, in particular the pressurizing unit, is adapted for reversibly everting a collapsed internal section of the sheath such as to become an expanded external section of the sheath at the distal annular end, such that a length of the middle section increases in a longitudinal direction of the tubular sheath.

The insertion instrument comprises at least one, in particular two, preferably three, of the following sensors: a shape sensor for detecting a shape of at least a part of the sheath, a position sensor for detecting a spatial position of at least a part of the sheath relative to the cavity, and an environment sensor for detecting a distance of a part of the sheath relative to the cavity or for detecting a property of the cavity.

This/these sensor(s) is/are configured for acquiring status data of the sheath and/or the cavity.

The cavity can be a lumen of the patient, in particular a gastrointestinal or vascular lumen of the patient.

Status data as understood herein can be raw data or processed data, i.e. can comprise more complex processed data, e.g. acquired raw status data which was processed by a processing unit or a microprocessor of the sensor to reconstruct status data, or directly measured status data via the sensors as e.g. physical values such as the resistance or a capacitance.

In alternative or addition to the pressurizing unit, the insertion instrument may comprise a driving unit for engaging, in particular frictionally engaging, the collapsed internal section of the tubular sheath for advancing the internal section in a distal and proximal direction for everting the sheath. The driving unit may be arranged at or within the proximal annular end and/or distal annular end (for details on the eversion mechanism see e.g. WO 2022/057296 A1).

Detection of the shape, the position, the distance of a part of the sheath and/or a property of the cavity by the sensors allows for enhanced, precise positioning and localization of the insertion instrument with respect to the cavity.

A property of the cavity may for example be the shape, in particular a constriction or deformation of the cavity, a tissue type of the detected environment, e.g. the mucosa tissue, muscle tissue, or tumor tissue, or a location, e.g. a specific location within the esophagus, the duodenum, the jejunum, or the ileum of a patient or for example the specific location of a calcification or an aneurism.

The at least one sensor can be arranged on a non-evertible internal section, in particular within a lumen, of the sheath or on the evertible internal section which is evertible such as to become the expanded external section.

Placing the sensor(s) on the internal section of the sheath allows the sensor(s) from the internal section to be transferred from the distal end to the external section during eversion of the sheath or vice versa.

The tubular sheath can be made of or comprise polyethylene, polyamide, polyurethane, or other thermoplastic elastomers. These materials are sufficiently biocompatible and flexible for deformation of the tubular sheath corresponding to a convoluted cavity, e.g. an intestinal cavity.

The sensor or at least one measuring section of the sensor may be arranged at a specific section or at a plurality of specific sections of the tubular sheath which have a specific distance from the proximal annular end in an everted configuration of the tubular sheath. Moreover, the sensors or the measuring section(s) may be arranged at a specific circumferential position of the tubular sheath. This allows sensors to be placed at a specific distance from the proximal annular end and/or a specific circumferential region of the cavity, such as a region of interest within patient's gastrointestinal tract, that is of relevance for the intervention, e.g. for deployment of an implant or for performing a surgical procedure.

At least one sensor can be deformable in unison with the sheath upon a deformation of the sheath, in particular in a radial direction, a circumferential direction, and/or the longitudinal direction of the sheath.

If the sensor is deformable in unison upon a deformation of the sheath, the sheath can continue to adapt to the shape of the cavity/environment, especially the gastrointestinal tract, despite comprising the sensor. The sensor being deformable with the flexible tubular sheath further prevents, that forces are exerted on the cavity which may cause trauma. In addition, fewer shear stress is generated within the surgical instrument during deformation.

At least one of the sensors can be a capacitive sensor which comprises at least two electrically conducting members which are separated from each other by a dielectric material. The conducting members are connected or connectable to a, preferably external, voltage source.

The capacitive sensor is configured for measuring a capacitance between the at least two conducting members indicative of a proximity to a surrounding tissue. The capacitive sensor is configured for acquiring status data in form of a length of at least a section of the sheath, in particular the middle section in the longitudinal direction of the sheath.

In addition or alternatively, the capacitive sensor can be configured for acquiring status data in the form of a curvature of the cavity and/or an obstruction, in particular calcifications or constrictions, radially and/or distally relative to the expanded external section of the sheath.

In addition or alternatively, the capacitive sensor is configured for acquiring status data in form of the shape of at least the part of the sheath.

A processing unit can be associated with the insertion instrument, in particular with or as a part of the sensors of the insertion instrument. The processing unit can be adapted to determine the shape of the cavity, the curvature of the cavity and/or an obstruction, and/or the shape of at least the part of the sheath via the capacitive sensor,measurements.

The total capacitance of the capacitive sensor can result from the sum of a constant capacitance c₀ between the at least two conducting members and a capacitance c₁ which is dependent on the environment of the capacitive sensor.

Two or three of the capacitive sensors, the resistive sensor and the optical fiber sensor can be used jointly to determine the curvature and position of the insertion instrument more precisely and reliably. The capacitive sensor may e.g. detect a deformation of the insertion instrument and the precise curvature of the deformation, but a spatial direction of the curvature may be determined via the optical fiber sensor.

The processing unit may be adapted to process the raw status data of the two or three of the above sensors to processed status data jointly.

The constant capacitance c₀ results from the constant distance and geometry of the conducting members and a permittivity of the dielectric material between the conducting members.

The total capacitance is dependent on the environment changes in accordance with the dielectric coefficient ε_{R} of the environment through which the electric field of the capacitive sensor extends. Therefore, the capacitance changes with a distance of the capacitive sensor with respect to the environment, in particular the cavity. Since walls of the cavity typically have a different dielectric coefficient ε_{R} than a medium within the cavity, the curvature of the cavity and/or obstructions can be reconstructed from the measured capacitance, e.g. by the processing unit which is connected/connectable to the capacitive sensor.

Moreover, a portion of the capacitive sensor which is located on or within the internal section of the tubular sheath typically has a lower capacitance due to the larger distance to the tissue than the portion of the capacitive sensor which is located on or within the expanded outer section. Consequently, the status data acquired by the capacitive sensor can be used for reconstructing the length of sections of the sheath, in particular the middle section.

Furthermore, based on deformations of the sheath and consequently the at least one capacitive sensor, the geometry and spacing between the at least two, in case of more than one capacitive sensor at least four conducting members, changes which changes the capacitance measured via the capacitive sensor(s). Based on the capacitance changes, the complete shape or the shape of the part of the sheath can be reconstructed, e.g. by the processing unit connected or connectable to the capacitive sensor(s).

The processing unit can be adapted for carrying out an error correction of the processed status data based on error margins associated with the corresponding sensor or corresponding sensors. The error margins of the corresponding sensor, e.g. the optical fiber sensor, the capacitive sensor, or the resistive sensor, can be defined, e.g. by using confidence intervals, in particular based on tests performed under laboratory conditions.

The processing unit can further be adapted to carry out the error correction of two sensors based by weighing the corresponding error margins of the sensors against each other so that a probable shape/position of the insertion instrument can be determined by the processed status data.

For example the status data detected by the optical fiber sensor via fiber Bragg gratings can be enhanced by processing using the error correction based on the error margins of the optical fiber sensor and the error margins of an additional sensor, in particular the capacitive or resistive sensor.

Thus, the accuracy of shape and position determination can be improved and e.g. machine learning procedures for shape/position reconstruction may be enhanced.

At least a first section of the conducting member may be arranged in a first orientation, in particular in the longitudinal direction of the sheath, and at least a second section of the conducting member may be arranged in a second orientation different from the first orientation, in particular in a circumferential direction of the sheath.

The orientation may correspond to a uniform orientation, such as an at least partially circular, helical, or rectilinear orientation, in particular with respect to the sheath.

Arranging sections of the conducting member in different orientations, in particular in the longitudinal and/or circumferential direction, maximizes the surface area of the conducting member and therefore optimizes the acquisition of status data indicative of the length of the middle section, the curvature of the cavity/the obstruction and the complete or partial shape of the tubular sheath.

The conducting members can comprise multiple interengaging projections which are arranged at least partially parallel to each other. The interengaging projections allow the adjacent area of conducting members to be maximized and the average spacing between the conducting members can be decreased such that a higher capacitance can be achieved.

The conducting members, in particular the interengaging projections, can be separated from each other by a thin dielectric material, in particular a dielectric surface coating.

At least one of the sensors can be formed by at least two, in particular a plurality, of electrodes.

The insertion instrument can additionally or alternatively comprise at least two, preferably a plurality of electrodes configured to contact a tissue for classification of a tissue type and preferably an impedance analyser conductively connected to the electrodes.

The impedance analyser may be configured for measuring the complex impedance dependent on a temporally varying frequency of an input applied to the electrodes to determine a dielectric behaviour e.g. of biological tissue contacting the electrodes. The impedance analyser may be integrated into the processing unit or formed by a separate unit.

The classification of the tissue type allows for identifying specific pathologies of tissue and/or target sites within the cavity.

The electrodes may be arranged at a specific circumferential or longitudinal portion of the sheath. The insertion instrument may comprise more than two electrodes which are spaced apart from each other in the circumferential and/or the longitudinal direction of the tubular sheath.

The impedance analyser may be configured to carry out measurements with a plurality of different pairs of electrodes.

At least one of the sensors can be a resistive sensor which is configured for acquiring status data in the form of mechanical displacement data based on the changes in resistance of at least a part of the sheath. Alternatively or additionally, the resistive sensor is configured for acquiring status data in the form of a displacement force and/or of at least the shape of a part of the cavity, in particular based on the mechanical displacement data.

The resistive sensor may be formed by at least one strain gauge, in particular a plurality of strain gauges, which is known to the person skilled in the art. The strain gauge or the plurality of strain gauges may comprise at least one Wheatstone bridge respectively such that the accuracy/reliability of the electric resistance measurement via the strain gauge can be increased. The status data in the form of mechanical displacement of the sheath, the displacement force exerted on the sheath and/or at least the shape of the part of the cavity can e.g. be used for identifying constrictions of the cavity and/or prevent potentially harmful/damaging pressure spikes exerted on the cavity walls/the insertion instrument.

The resistive sensor can be formed by at least one conductive layer, in particular multiple conductive layers, arranged at least partially on or in the sheath, e.g. by printing of a conductive material.

Arranging the conductive layer on or in the sheath, such that the conductive layer can be arranged on the expanded external section of the sheath, allows for an improved and sensitive measurement of (i) the mechanical displacement of the sheath, (ii) the displacement force exerted on the sheath, and/or (iii) the shape of at least a part of the cavity.

The resistive sensor can comprise a plurality of connected sections with different orientations relative to each other, in particular at least two sections which are perpendicular with respect to each other.

At least a section of the resistive sensor can be arranged in the longitudinal direction of the sheath and at least another section can be arranged in a circumferential direction of the sheath.

This arrangement of the resistive sensor maximizes the surface area of the resistive sensor to optimize acquiring the status data indicative of the mechanical displacement, the displacement force, and/or the shape of the cavity.

The resistive sensor can have at least one measuring segment, in particular multiple measuring segments. In the at least one measuring segment, the resistive sensor extends in a plurality of parallel, but non-contacting, zig-zac-like windings.

By arranging the resistive sensor/conductive layer in windings in the measuring segment allows for a more sensitive measurement within the measuring segment which may be configured to be positioned in a region of interest, e.g. the target site.

The windings can be separated from each other by a thin insulating material, in particular an insulating surface coating.

The measuring segment(s) can extend only over a partial circumferential and/or longitudinal region of the sheath, such that the resistive sensor is configured for enhanced acquisition of status data within the measuring section(s).

In one embodiment of the insertion instrument at least two resistive sensors simultaneously function as the capacitive sensor of the insertion instrument.

One of the sensors can comprise or consist of a conductive material, in particular copper, gold, aluminium, silver, or a carbon material.

These materials have good conductive properties and can be applied to the sheath in thin deformable films which can deform in accordance with an elastic substrate, in particular the elastic tubular sheath, on or in which they can be deposited.

The shape sensor and/or the position sensor can be or include an optical fiber sensor which has a fiber Bragg grating for acquiring status data in the form of three-dimensional shape data of, a strain and/or a force on at least a part of the sheath by a wavelength dependent transmission and reflection of light. The optical fiber sensor is arranged on or in the sheath and extends at least partially between the proximal annular end and the distal annular end or the optical fiber sensor is located on a separate guidewire of the insertion instrument (see e.g. US 9,404,734 B2 for shape sensing integration via an optical fiber sensor).

If the optical fiber sensor is arranged on the sheath, the optical fiber sensor can be evertible from the internal section to the expanded external section and vice versa.

However, insertion instruments with a small cross-sectional diameter would require a deformation of the optical fiber sensor at a sharp bend, in particular at the distal end when everting the sheath. In order to reduce the bend angle, the optical fiber can extend at least partially in the circumferential direction, in particular helically to the longitudinal axis.

The optical fiber sensor can be located on a separate guidewire of the insertion instrument and the insertion instrument can comprise a guidewire lumen for insertion of the guidewire. The guidewire lumen can have an opening at the proximal annular end and extend along the sheath up to the distal annular end of the sheath. The lumen may have a closed end at the distal annular end of the sheath or form a distal opening.

The guidewire comprising the optical fiber can be advanceable within the lumen, preferably fully automatically, controlled by the processing unit, optionally via an advancement actuator. The guidewire can be positioned in accordance with an everting state of the sheath, e.g. such that a distal tip of the guidewire can always be positioned at the distal annular end.

The insertion instrument can comprise a data transmission unit for wired or wireless data transmission of the acquired status data. In addition, the insertion instrument preferably has a power supply which is arranged internally within the sheath, in particular at the data transmission unit.

The data transmission unit may include D/A or A/D converters, high and low pass filters, or other electronic components for processing, transmitting, and receiving data known to the skilled person.

The data transmission unit can be connected via a wire or wirelessly to the processing unit which is arranged externally from the cavity. Thus, the steps for reconstructing the shape, position, and/or environment based on the unprocessed status data that may be computationally demanding can be performed externally from the insertion instrument.

An internally arranged power supply, in particular at the data transmission unit and/or a wireless data transmission unit reduces the number of electric lines of the insertion instrument and reduces interference between the electric components of the insertion instrument.

At least one of the sensors configured for acquiring status data of the sheath and/or cavity can be an ultrasound sensor.

The insertion instrument can comprise the ultrasound sensor located on the distal annular end so that the ultrasound sensor remains stationary on the distal annular end, independent of the everting state of the sheath. The ultrasound sensor is configured for detecting a two-dimensional and/or three-dimensional image of the cavity.

The ultrasound sensor remaining stationary at the distal annular end allows for flexible adjustment of a position of the ultrasound sensor with respect to the cavity and to acquire local images of the cavity, in particular along a distal direction of the distal annular end, when advancing the insertion instrument. The ultrasound sensor may be arranged stationary attached on a distal annular end, in particular a distal head piece. The stabilizing member may be configured to retain a lumen of the insertion instrument in an open position such that e.g. surgical instruments may be advanced along the lumen with reduced friction as disclosed in WO 2022/057296 A1 or EP application number 21315286.1.

Alternatively or in addition to the ultrasound sensor, the insertion instrument may comprise an optical sensor, in particular an optical sensor, in particular a camera.

A system according to the invention has a previously described insertion instrument and a processing unit operatively connected to or comprised by the at least one sensor for processing the acquired raw status data to processed status data to (i) reconstruct the shape of at least a part of the tubular sheath, (ii) determine the spatial position of at least a part of the tubular sheath relative to the cavity, (iii) determine the distance of a part of the tubular sheath relative to the cavity or (iv) for detecting a property of the cavity. The processing unit can be adapted to reconstruct the length of at least a part of the tubular sheath, in particular the middle section.

The raw status data acquired by the sensor can be processed by the processing unit and used to control operation of the instrument via a closed feedback loop such that the determination/reconstruction of (i) - (iv) can be carried out automatically. The processing unit can be adapted to continuously augment the processed status data, e.g. a three-dimensional model of the tubular sheath and/or cavity, by processing raw status data acquired via the sensor(s) in the closed feedback loop.

The closed feedback loop can be fully automated so that no user input is required after the closed feedback loop is initiated. The processing unit can be adapted to generate control instructions for actuators, e.g. actuators for controlling the pressure within an inflatable chamber, a deflection of the insertion instrument, sensor positioning, such as the position of the optical fiber sensor in a lumen, based on the acquired status data. The processing unit may further be adapted to control the operations of the sensor(s), in particular by supplying an electric signal of the resistive or capacitive sensor or a light signal of the optical fiber sensor.

A process for manufacturing a previously described insertion instrument according to the invention be achieved by extrusion of the flexible tubular sheath, blow molding, in particular extrusion blow molding or injection blow modling, of the flexible tubular sheath, or additive manufacturing of the flexible tubular sheath.

The invention is now described with reference to certain embodiments and figures which show:
Figure 1: a semi-transparent representation of a system of a first embodiment of the insertion instrument having an optical fiber sensor;
Figures 2A and 2B: a schematic illustration of a first embodiments of a capacitive sensor placed adjacent a tissue and a second embodiment of a capacitive sensor having a plurality of interengaging projections;
Figure 3: a schematic illustration of a resistive sensor;
Figures 4A to 4C: a schematic illustration of an insertion instrument being advanced through a cavity;
Figures 5A to 5H: different embodiments of sensors/sensor designs of the insertion instrument;
Figures 6A to 6D: four different embodiments of a printed circuit assembly of a resistive sensor, a capacitive sensor, and electrodes.

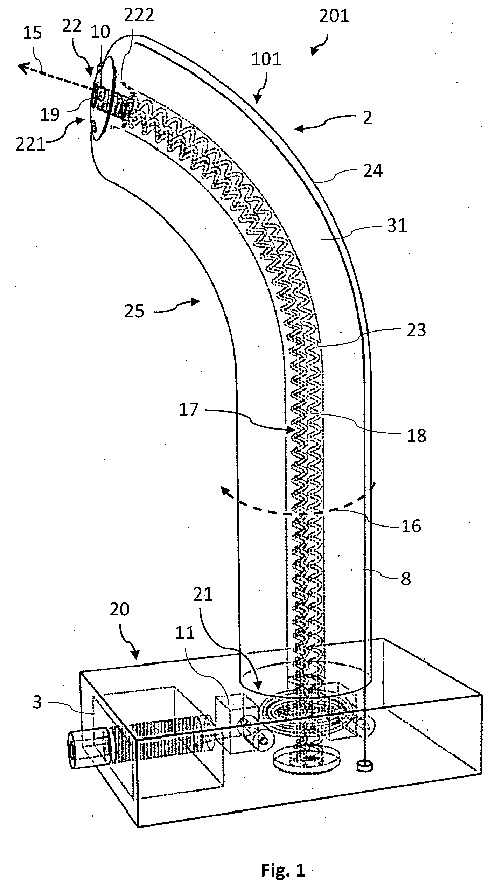

Figure 1 shows a first embodiment system 201 in a semi-transparent representation. The system has an insertion instrument 101 which forms a soft growing robot. The insertion instrument 101 has a flexible tubular sheath 2 which is connected to a proximal annular end 21 and has a middle section 25 which extends up to a distal annular end 22. At the distal annular end 22, a collapsed internal section 23 can be everted radially outward to become an expanded external section 24.

At the distal annular end 22 the insertion instrument 101 has a head piece 221 which has a guiding surface 222 for sliding along the tubular sheath 2 during eversion of the tubular sheath 2 such that the head piece 221 is stationary at the distal annular end 221 irrespective of the everting state of the insertion instrument 101. Furthermore, an ultrasound sensor 10 is mounted fixedly attached to the headpiece 221 with a field of view directed into a distal direction 15 of the insertion instrument 101.

The tubular sheath 2 is made of a polyethylene and has a central lumen 17. Within the central lumen 17 is a stabilizing member 18 which has three elastic springs that extend over the entire length of the lumen 17. Three channels are arranged in an axial direction of the insertion instrument 101 within the three elastic springs to convey surgical instruments from proximal openings to a common distal opening 19 of the channels. The springs are configured to retain the channels in an open configuration.

At the proximal annular end 21 the tubular sheath 2 is connected along its circumferential direction 16 to a proximal housing 20 which houses a pressurizing unit in form of a pump 3. The pump 3 is fluid-connectable to a pressure tight chamber 31 between the expanded external section 24 and the internal section 23.

In a different embodiment, the pressure tight chamber 31 may be arranged only at a distal annular end 22 and is sealed off from the rest of the enclosed volume of the tubular sheath 2, such that no unnecessary'frictional force is exerted on a surrounding of the insertion instrument 101, e.g. along the complete middle section 25 (see e.g. WO 2022/057296 A1 or EP application number 21315286.1).

Furthermore, Fig. 1 shows a processing unit 11 which is arranged within the housing 20 for controlling the insertion instrument 101 by de-/inflation of the pressure tight chamber 31 via the pump 3.

An optical fiber sensor 8 arranged on a guidewire 81 which is movable with respect to the tubular sheath 2 within a lumen of the tubular sheath 2 is further operatively connected to the processing unit 11. The processing unit 11 is configured to reconstruct the three-dimensional shape of the insertion instrument 101 based on the status data of the optical fiber sensor 8. Moreover, the processing unit 11 is configured for controlling an advancement/retraction of the guidewire 81 corresponding to an everting or bending state of the insertion instrument 101 such that a distal tip of the guidewire 81 can be located at the distal annular end 22.

However, the processing unit 11 need not be formed by a single unit disposed in the housing 20 of the system 201. In particular, the processing unit 11 can be divided into many processing subunits and/or at least partially based on cloud-based processing unit/processing subunits.

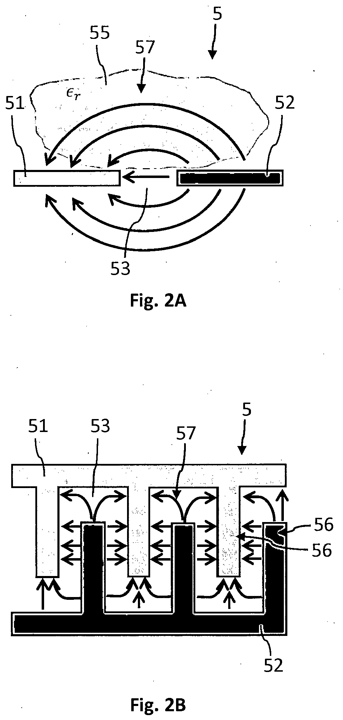

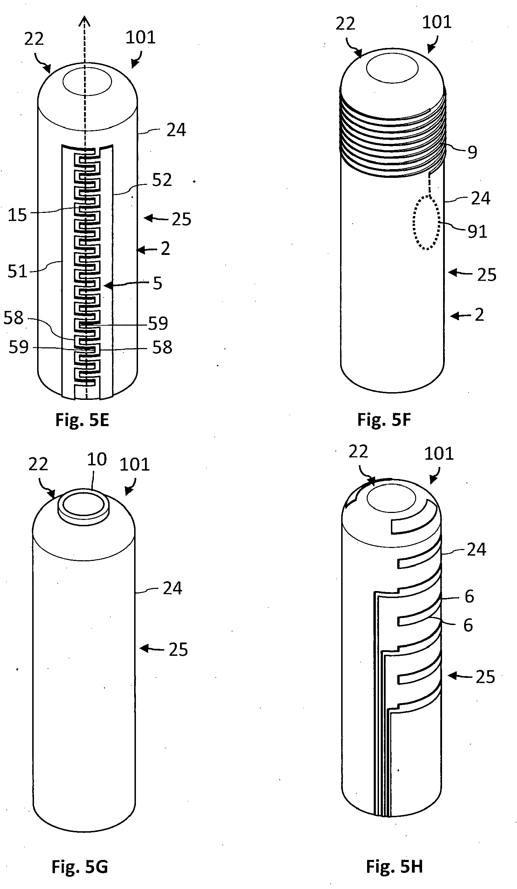

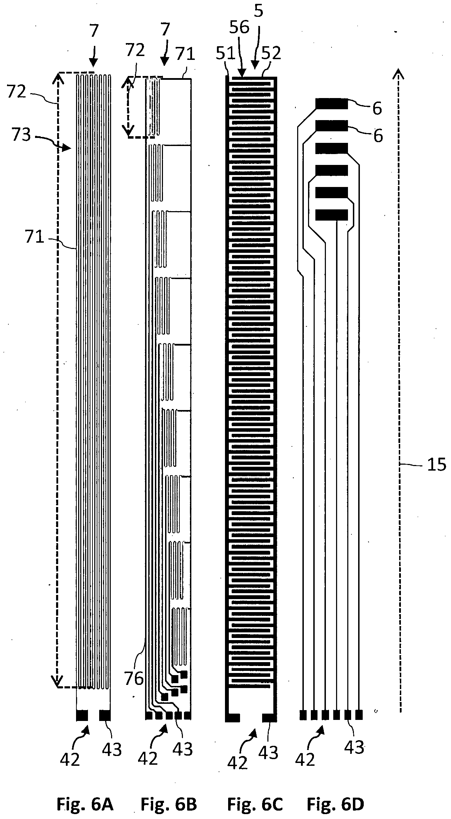

Figures 2A and 2B shows a schematic illustration of a first and second embodiment of a capacitive sensor 5 having a first conducting member 51 and a second conducting member 52 forming an electric field 57 therebetween due to an applied voltage difference between the conducting members 51, 52. A dielectric material 53 is arranged between the adjacent conducting members 51, 52 in Figs. 2A and 2B such that the conducting members 51, 52 are insulated from each other. The dielectric material 53 may alternatively only be applied as a surface coating onto the conducting members 51, 52 respectively. The embodiments of the capacitive sensor 5 of Figs. 2A and 2B may be placed on or within a tubular sheath of an insertion instrument (see e.g. Fig. Fig. 5E and Fig. 6C).

Fig. 2A shows that the capacitive sensor 5 is partially surrounded by a tissue 55 which has a different relative permittivity ε_{R} than bodily fluids, e.g. digestive fluids or blood, and a fluid within a pressure tight chamber of the insertion instrument. Therefore, the capacitance of the capacitive sensor 5 changes with its proximity to the surrounding tissue 55 and with the positioning of the capacitive sensor 5.

If the capacitive sensor 5 is, for example, disposed within an internal section 23 of a tubular sheath 2 of the insertion instrument 101 it is essentially only surrounded by the fluid within the pressure tight chamber 31 (see Fig. 1) and not by tissue, such that the capacitance is lower.

In contrast, if the capacitive sensor 5 is arranged on the expanded external section 24 it may be arranged adjacent the surrounding tissue of a patient, such that the capacitance is increased because the relative permittivity of tissue is typically greater than one, ε_{R} > 1.

Hence, the change in capacitance acquired by the capacitive sensor 5 can be used for determining the everting state of the sheath, in particular the length of a middle section of the sheath. The capacitive sensor can further be used of reconstruction of the shape of the tubular sheath, a spatial position/distance of the tubular sheath with respect to a cavity, or a property of the cavity.

The capacitive sensor 5 can be mechanically connected to the flexible tubular sheath 2 (see Fig. 1), e.g. sputtered on the tubular sheath 2 forming thin layered conducting members 5. By deformation of the sheath changes the distance between the conducting members 5 or changes of the surrounding capacitive medium, e.g. position/shape of surrounding tissue, the capacitance between the conducting members 5 changes which allow for reconstruction of the deformation/surrounding capacitive medium.

A processing unit (not shown in Figs. 2A and 2B) connected to the capacitive sensor 5 is configured to determine the length of a middle section of the tubular sheath which is dependent on the everting state of the tubular sheath, the spatial positions and shape of surrounding tissue 55 of the tubular sheath, and the relative position of the tubular sheath with respect to the surrounding tissue 55 based on capacitance changes measured via the capacitive sensor 5. The capacitive sensor 5 may also be used for classification of the surrounding tissue based on the measured changes in capacitance due to different relative permittivities of the surrounding tissues.

Figure 2B further shows a capacitive sensor 5 with first and second conducting members 51, 52 forming interengaging projections 56. The interengaging projections 56 increase the maximum capacitance sensitivity measured by the capacitive sensor 5 by maximizing an adjacent region/area of the conducting members 51, 52.

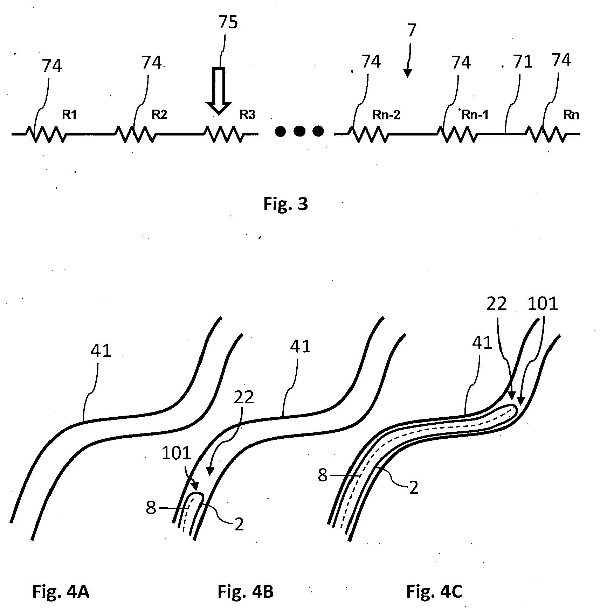

Figure 3 is a schematic illustration of a resistive sensor 7 with one conducting layer 71 having a plurality of resistors 74, R1 - Rn serially connected and equidistantly spaced apart from each other.

The resistive sensor 7 in Fig. 3 is connected to a processing unit such that a change of an electric signal indicative of a mechanical displacement force 75 exerted onto the resistive sensor 7 and a deformation of a tubular sheath of the insertion instrument on which the resistive sensor 7 is deposited can be detected. The resistive sensor 7 comprises multiple resistors 74 or a specific materially dependent resistance such that a force exerted on the sheath causes strain which can be measured with the resistive sensor 7 by way of a change in electrical resistance.

If the resistive sensor 7 is stretched without breaking/inelastic deformation the conducting layer 71 becomes longer and narrower such that its electrical resistance from end to end is increased. If the If the resistive sensor 7 is compressed, the conducting layer 71 becomes shorter and broader which decreases its electrical resistance from end to end.

The processing unit is configured to measure a resistance, and to reconstruct based thereon mechanical displacement data, a displacement force, and a stretch of the insertion instrument based on the displacement force. Thereby, the shape of the tubular sheath can be reconstructed by the processing unit via the collected data of the resistive sensor 7.

In a different embodiment of the resistive sensor 7, specific segments of the resistive sensor 7 comprise no resistors/little material dependent resistance 74 so that forces exerted on this section have only a minor influence on the measured resistance. Instead, the resistive sensor 7 has a dedicated measuring segment in which electrical resistors are arranged at a small distance from each other, so that forces exerted on this measuring segment can be detected particularly sensitively.

In addition, the conducting layer 71 in the measuring segment 72 can be arranged on or in a tubular sheath in an area maximized by windings 73 or other surface structures (see e.g. Fig. 4C). This can also increase the measurement sensitivity of the resistive sensor 7 in the respective measuring segment.

Alternatively, to separately formed resistors 74 as shown in Fig. 3, the electrical resistance of the resistive sensor 7 may be based on the material dependent resistance of a material or multiple materials making up the conducting layer 71.

Figures 4A to 4C show a schematic illustration of an insertion instrument 101 being inserted through an intestinal cavity 41 of a patient. The insertion instrument 101 has an optical fiber sensor 8 arranged in a central lumen of the insertion instrument 101 which is advanced together with the insertion instrument 101. The insertion instrument 101 is advanced through the insertional cavity 41 by a flexible tubular sheath 2 being everted from a distal annular end 22 of the insertion instrument 101 (see Fig. 1). The optical fiber sensor 8 is connected to a processing unit (not shown in Figs. 4A - 4C) which is configured to reconstruct the shape of the insertion instrument 101 based on the wavelength dependent reflection/transmission of a light signal transmitted through the optical fiber sensor 8.

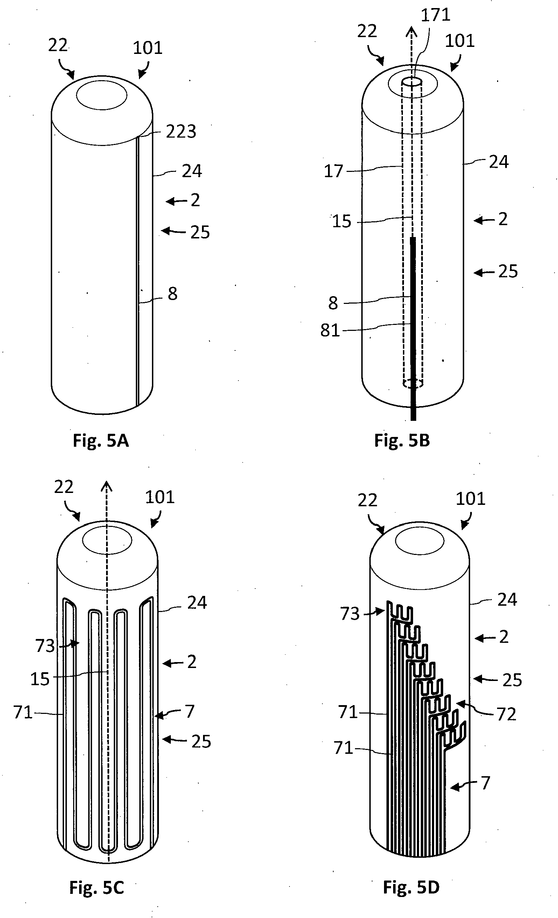

Figures 5A to 5H shows the various embodiments of the insertion instrument 101 in the form of an evertible insertion instrument 101 with different sensors 5, 7, 8/sensor designs. The insertion instrument 101 is evertible such that an internal section (not shown in Figs. 5A - 5H) becomes an expanded external section 24 of a flexible tubular sheath 2. A processing unit 11 is configured to feed or draw pressurized fluid into an internal pressure tight chamber 31 of the insertion instrument 101 (see Fig. 1) such that a length of a middle section 25 of the insertion instrument 101 can be controlled.

Figure 5A shows the first embodiment of the insertion instrument 101 (see Fig. 1) which has an optical fiber sensor 8 which is arranged within a lumen of a tubular sheath 2 of the insertion instrument 101 and is movable with respect to the tubular sheath 2. The fiber sensor 8 can be automatically advanced based on a signal input of the processing unit, in accordance with the everting state of the insertion instrument 101. Based on the measurements of the optical fiber sensor 8, the current shape of the insertion instrument 101 can be reconstructed via the processing unit. The internal lumen further has a closed distal end 223 at the annular end 22, such that the fiber sensor 8 cannot be advanced too far within the lumen.

Figure 5B shows a second embodiment of the insertion instrument 101 with a central internal lumen 17 with a distal opening 171, shown by the dashed line in Fig. 5B. The internal lumen 17 is held in an open state, e.g. by a stabilizing member, in form of springs (see Fig. 1), such that a guidewire 81 carrying the optic fiber sensor 8 can be advanced with minimal frictional resistance along the internal lumen 17, e.g. in a distal direction 15 of the insertion instrument 101.

Figures 5C and 5D show a third and fourth embodiment of the insertion instrument 101 comprising a resistive sensor 7 arranged on the flexible tubular sheath 2 of the insertion instrument 101.

The resistive sensor 7 in Fig. 5C has a conductive layer 71 which forms a plurality of parallel windings 73 which are oriented along a longitudinal direction 15 of the insertion instrument 101 parallel to each other. The conductive layer 71 is formed by a thin metal film which is deposited on the tubular sheath 2 and covered by an insulating film such that they are elastically deformable in unison with a deformation of the tubular sheath 2. Since the conductive layer 71 extends over a complete circumference of the sheath 2, a displacement force/deformation of the tubular sheath 2 of the insertion instrument 101 can be detected due to also being exerted on the resistive sensor 7.

Alternatively in a different embodiment of the insertion instrument 101, a plurality of resistive sensors 7 may be arranged at different specific circumferential sections of the sheath 2 such that the change in resistance due to a deformation measured by a resistive sensor 7 can be attributed to the specific circumferential section.

The plurality of resistive sensors 7 in Fig. 5D respectively comprise a conductive layer 71. The plurality of conductive layers 71 extend parallel to each other from a proximal annular end (not shown in Fig. 5D) along the longitudinal direction 15. Each conductive layer 71 leads to a measuring segment 72 at a distal end of the conductive layer 71. At the measuring segment 72 the conductive layers 71 form multiple windings 73 such that the surface area and therefore the sensitivity for detecting a deflecting force/deformation in a region of the measuring segment 72 is increased.

A processing unit (not shown in Figs. 5C and 5D) is connected to the plurality of conductive layer(s) 71 of the resistive sensors 7 such that a resistance of the conducting layers 71 can be measured. The conducting layers 71 can comprise sections with different electric resistances, in particular resistors (see e.g. Fig. 3). The processing unit is further configured to reconstruct a deformation force/deformation on the windings 73/measuring segment 72 of the tubular sheath 2 via the resistive sensors 7 and the corresponding three-dimensional shape of the insertion instrument 101 at the windings 73/measuring segment 72.

Figure 5E shows a fifth embodiment of the insertion instrument 101 which has a capacitive sensor 5 which has a first conducting member 51 and a second conducting member 52. The conducting members 51, 52 are arranged in a serpentine pattern which is offset relative to each other so that one wide turn 58 of both conducting members 51, 52 always frames a narrow turn 59 of the other conducting member 51, 52: In this embodiment, the wide turn 58 extends further along a longitudinal direction 15 of the insertion instrument 101 than the small turn 59.

The capacitive sensor 5 can be operated in combination with a processing unit for reconstruction of shape of the tubular sheath, a spatial position/distance of the tubular sheath with respect to a cavity, or a property of the cavity, in a similar manner as described in Fig. 2A and Fig. 2B.

Figure 5F shows a sixth embodiment of the insertion instrument 101 having a wireless data transmission unit in form of an antenna 9 connected to an internally arranged power supply 91 depicted by dashed lines. The antenna 9 is configured for wirelessly transmitting and receiving radio frequency signals, such as input commands from a processing unit or acquired status data of the tubular sheath 2/cavity. The antenna 9 may optionally only be configured for transmitting the radio frequency signals and not receiving radio frequency signals.

The processing unit is connected via a wireless interface to the antenna 9 such that return lines of the corresponding sensors (not shown in Fig. 5F) are not required. This minimizes an interference between sensors/lines. The processing unit is configured for receiving the status data wirelessly transmitted by the antenna 9 and optionally further configured to wirelessly send control commands to the antenna 9 based the received and processed status data. The antenna 9 is electrically connected to the respective sensors, e.g. the resistive/capacitive/electrode sensors 5, 7, 8 described'in Fig. 5A to Fig. 5E, for acquiring the status data. The antenna 9 further has an A/D converter and optionally a D/A converter and respective reconstruction filters for converting the quantified information to binary information and optionally vice versa. This can allow the status data to be transmitted and the control commands to be received via the antenna 9.

Fig. 5F shows that the antenna 9 is arranged on an expanded external section 24 of a tubular sheath 2 of the insertion instrument 101. However, the antenna 9 may alternatively be arranged internally within the insertion instrument 101 or e.g. attached to a head piece 221 stationarily located at the distal annular end 22 (see Fig. 1).

Figure 5G shows a seventh embodiment of the insertion instrument 101 with an ultrasound sensor 10 attached at a distal annular end 22 of the insertion instrument 101. The ultrasound sensor 10 is arranged on a head piece 221 with a guiding surface 222 (see Fig. 1) such that it is positioned at the distal annular end 22 irrespective of an everting state of the insertion instrument 101. A processing unit which is connected to the ultrasound sensor, e.g. via an antenna 9 of Fig. 5F, is configured to reconstruct a three-dimensional model of the cavity which is augmented continuously by advancing the insertion instrument 101 into the cavity and local imaging by the ultrasound sensor 10.

Figure 5H shows an eighth embodiment of the insertion instrument 101 which has a plurality of pairs of electrodes 6 forming an impedance sensor which is connected to a processing unit which serves as an impedance analyzer. The electrodes 6 are arranged equidistantly spaced apart from each other in an axial direction of the insertion instrument 101. The processing unit is adapted to classify the tissue contacted by the impedance sensors based on the measured impedance of the respective contacted tissue.

Figures 6A to 6D show four different embodiments of a printed circuit assembly of different versions of the resistive sensor 7, the capacitive sensor 5, and electrodes 6 for the impedance analyzer. The printed circuit assembly can be applied to a flexible tubular sheath of an insertion instrument to form the sensor. At a proximal end 42 of the printed circuit assembly there are a plurality of contacts 43 which are connectable to a processing unit for connecting the sensor 7, 5 or electrodes 6. The printed circuit assembly is formed by depositing a thin metal layer on a flexible tubular sheath of the insertion instrument.

Figure 6A shows a printed circuit assembly of a resistive sensor 7 with a conductive layer 71 which forms a plurality of windings 73 arranged in parallel to a longitudinal direction 15. The plurality of windings 73 forms a longitudinally extended measuring segment 72 of the resistive sensor such that a deformation of the measuring segment 72 may be detected via a processing unit.

Figure 6B shows a different embodiment of a printed circuit assembly forming a resistive sensor 7 which has multiple measuring segments 72 formed by conductive layers 71, 76 according to Fig. 6A arranged in a row along the longitudinal direction 15. A first common conductive layer 71 is connected to each measuring segments 72 and a respective second conductive layer 76 extends back along from each measuring segment 72 to respective contacts 43 at the proximal end 42 of the printed circuit assembly. The resistive sensor 7 of Fig. 6B therefore allows to measure a deformation of each measuring segment 72 independently from each other. The resistive sensor 7 of Fig. 6B may be arranged along specific circumferential sections of the insertion instrument, such that a plurality of resistive sensors 7 of Fig. 6B can be used for reconstructing a three-dimensional shape of the insertion instrument.

Figure 6C shows an embodiment of a printed circuit assembly of a capacitive sensor 5 extending e.g. along the longitudinal direction 15 of a tubular sheath with a first conducting member 51 and a second conducting member 52. The conducting members 51, 52 form interengaging projections 56 (see also Fig. 2B) which are arranged parallel to each, e.g. other along a circumferential direction of the tubular sheath.

Figure 6D shows an embodiment of a printed circuit assembly forming a plurality of electrodes 6 which are arranged equidistantly spaced apart from each other, e.g. along the longitudinal direction 15 of a flexible tubular sheath. Each electrode 6 is wired to a separate contact 43 for connecting to the processing unit. The processing unit is adapted for carrying out a measurement between two different electrodes 6 contacting a tissue of a patient. Thereby the processing unit can carry out a plurality of measurements corresponding.to (n - 1)! possible combinations of electrodes 6, n corresponding to the number of electrodes. Consequently, multiple electrodes 6 for contacting the tissue at different spatial positions can allow for enhanced classification of the tissue contacted by the electrodes 6.

## Claims

1. An insertion instrument (101), in particular a surgical insertion instrument (101), for insertion into a cavity (41), preferably a human cavity (41), the instrument (101) comprising
- a flexible tubular sheath (2) having a proximal annular end (21), a distal annular end (22), and a middle section (25) which extends between the proximal annular end (21) and the distal annular end (22),
- optionally a pressurizing unit (3) for pressurizing at least one inflatable pressure tight chamber (31) of the flexible tubular sheath (2), in particular at or within the distal annular end (22),
∘ the pressurizing unit (3) comprising a fluid supply which is fluid-connected or connectable to the inflatable chamber (31) for supplying a pressurized fluid,
- the insertion instrument (101), in particular the pressurizing unit (3), being adapted for reversibly everting a collapsed internal section (23) of the sheath (2) such as to become an expanded external section (24) of the sheath (2) at the distal annular end (22) such that a length (4) of the middle section (25) increases in a longitudinal direction of the tubular sheath (2),
**characterized in that** the insertion instrument (101) comprises at least one, in particular two, preferably three, of
- a shape sensor (5, 6, 7, 8, 10) for detecting a shape of at least a part of the sheath (2),
- a position sensor (5, 6, 7, 8, 10) for detecting the spatial position of at least a part of the sheath (2) relative to the cavity (41), and
- an environment sensor (5, 6, 7, 8, 10) for detecting a distance of a part of the sheath (2) relative to the cavity (41) or for detecting a property of the cavity (41),
which is/are configured for acquiring status data of the sheath (2) and/or the cavity (41).

2. Insertion instrument (101) according to claim 1, wherein the at least one sensor (5, 6, 7, 8, 10) is arranged on a non-evertible internal section, in particular within a lumen, of the sheath (2) or on the evertible internal section (23) which is evertible such as to become the expanded external section (24).

3. Insertion instrument (101) according to any one of the preceding claims, wherein the at least one sensor (5, 6, 7, 8, 10) is deformable in unison with the sheath upon a deformation of the sheath (2), in particular in a radial direction, a circumferential direction and/or the longitudinal direction of the sheath (2).

4. Insertion instrument (101) according to one of the preceding claims, wherein at least one of the sensors (5, 6, 7, 8, 10) is a capacitive sensor (5) which comprises at least two electrically conducting members (51, 52) which are separated from each other by a dielectric material (53) and the conducting members (51, 52) are connected or connectable to a, preferably external, voltage source,
wherein the capacitive sensor (5) is configured for measuring a capacitance between the at least two conducting members (51, 52) indicative of a proximity to a surrounding tissue (55), such that the capacitive sensor (5) is configured for acquiring status data in form of
- a length (4) of at least a section of the sheath (2), in particular of the middle section (25) in the longitudinal direction of the sheath (2),
- a curvature of the cavity (41) and/or an obstruction, in particular calcifications or constrictions, radially and/or distally relative to the expanded external section (24) of the sheath (2), and/or
- the shape of at least a part of the sheath (2).

5. Insertion instrument (101) according to claim 4, wherein at least a first section of the conducting member (51, 52) is arranged in a first orientation, in particular in the longitudinal direction (15) of the sheath (2), and at least a second section of the conducting member (51, 52) is arranged in a second orientation different from the first orientation, in particular in a circumferential direction (16) of the sheath (2).

6. Insertion instrument (101) according to one of the claims 4 or 5, wherein the conducting members (51, 52) comprise multiple interengaging projections (56) which are arranged at least partially parallel to each other.

7. Insertion instrument (101) according to any one of the preceding claims, wherein at least one of the sensors (5, 6, 7, 8, 10), is formed by electrodes (6) configured to contact a tissue for classification of a tissue type and preferably an impedance analyser conductively connected to the electrodes (6) .

8. Insertion instrument (101) according to any one of the preceding claims, wherein at least one of the sensors (5, 6, 7, 8, 10) is a resistive sensor (7) which is configured for acquiring status data in form of
- mechanical displacement data based on the changes in resistance of at least a part of the sheath (2),
- optionally a displacement force and/or at least the shape of a part of the cavity (41), in particular based on the mechanical displacement data.

9. Insertion instrument (101) according to claim 8, wherein the resistive sensor (7) is formed by at least one conductive layer (71), in particular multiple conductive layers (71), arranged at least partially on or in the sheath (2).

10. Insertion instrument (101) according to one of the claims 8 or 9, wherein at least a section of the resistive sensor (7) is arranged in the longitudinal direction (15) of the sheath (2) and at least another section is arranged in a circumferential direction (16) of the sheath (2).

11. Insertion instrument (101) according to one of the claims 8 to 10, wherein the resistive sensor (7) has at least one measuring segment (72), in particular multiple measuring segments (72), wherein the resistive sensor (7) extends in a plurality of parallel, but non-contacting, windings (73) in the at least one measuring segment (72).

12. Insertion instrument (101) according to one of the preceding claims, wherein one of the sensors (5, 6, 7) comprises or consists of a conductive material, in particular copper, gold, aluminium, silver, or a carbon material.

13. Insertion instrument (101) according to any one of the preceding claims, wherein the shape sensor (5, 6, 7, 8, 10) and/or the position sensor (5, 6, 7, 8, 10) is an optical fiber sensor (8) which has a fiber Bragg grating for acquiring status data in form of three-dimensional shape data, a strain and/or a force of at least a part of the sheath (2) by a wavelength dependent transmission and reflection of light, wherein
the optical fiber sensor (8) is arranged on or in the sheath (2) and extends at least partially between the proximal annular end (21) and the distal annular end (22) or the optical fiber sensor (8) is located on a separate guidewire (81) of the insertion instrument (101).

14. Insertion instrument (101) according to any one of the preceding claims, wherein the insertion instrument (101) comprises a data transmission unit (9) for wired or wireless data transmission of the acquired status data and
the insertion instrument (101) further preferably has a power supply (91) which is arranged internally within the sheath (2), in particular at the data transmission unit (9) .

15. Insertion instrument (101) according to any one of the preceding claims, wherein at least one of the sensors (5, 6, 7, 8, 10) is an ultrasound sensor (10) located on the distal annular end (22) so that the ultrasound sensor (10) remains stationary on the distal annular end (22), independent of the everting state of the sheath (2) and the ultrasound sensor (10) is configured for detecting a two-dimensional and/or three-dimensional image of the cavity (41).

16. A system (201) comprising an insertion instrument (101) according to one of the proceeding claims and further having a processing unit (11) operatively connected to or comprised by at least one sensor (5, 6, 7, 8, 10) for processing the acquired raw status data to processed status data to (i) reconstruct the shape, in particular length, of at least a part of the tubular sheath (2), (ii) determine the spatial position of at least a part of the tubular sheath (2) relative to the cavity (41), (iii) determine the distance of a part of the tubular sheath (2) relative to the cavity (41) or (iv) for detecting a property of the cavity (41).
